# EUROPEAN PATENT SPECIFICATION

(11) **EP 2 303 203 B1**
(45) Date of publication and mention of the grant of the patent: **20.06.2012**
(21) Application number: 09747269.0
(22) Date of filing: 11.05.2009
(51) Int. Cl.: A61F 9/007, A61B 17/32, A61B 17/34, A61B 17/30, A61M 11/00

(54) **SMALL GAUGE MECHANICAL TISSUE CUTTER/ASPIRATOR PROBE FOR GLAUCOMA SURGERY**
SCHMALE MECHANISCHE GEWEBESCHNEIDE/ASPIRATIONSSONDE FÜR GLAUKOM-OPERATIONEN
INSTRUMENT DE PETIT CALIBRE À BISTOURI MÉCANIQUE ET SONDE D'ASPIRATION POUR CHIRURGIE DU GLAUCOME

(30) Priority: 15.05.2008 US 120867
(43) Date of publication of application: 06.04.2011
(73) Proprietor: Alcon Research, Ltd., Fort Worth, Texas 76134 (US)
(72) Inventor: LIND, Casey, Orange CA 92869 (US); HUCULAK, John C., Mission Viejo CA 92692 (US)
(74) Representative: Hanna, Peter William Derek
(86) International application number: PCT/US2009/043420
(87) International publication number: WO 2009/140185

(56) References cited:
- EP-A- 0 537 116
- WO-A-03/045290
- WO-A-2007/121485
- US-A- 4 530 359
- US-A- 5 527 332
- US-A- 5 733 297

## Description

### BACKGROUND OF THE INVENTION

The present invention relates to glaucoma surgery and more particularly to a device for performing glaucoma surgery using a small gauge mechanical tissue cutter/aspirator probe with a retractable pick.

Glaucoma, a group of eye diseases affecting the retina and optic nerve, is one of the leading causes of blindness worldwide. Glaucoma results when the intraocular pressure (IOP) increases to pressures above normal for prolonged periods of time. IOP can increase due to an imbalance of the production of aqueous humor and the drainage of the aqueous humor. Left untreated, an elevated IOP causes irreversible damage the optic nerve and retinal fibers resulting in a progressive, permanent loss of vision.

The eye's ciliary body epithelium constantly produces aqueous humor, the clear fluid that fills the anterior chamber of the eye (the space between the cornea and iris). The aqueous humor flows out of the anterior chamber through the uveoscleral pathways, a complex drainage system. The delicate balance between the production and drainage of aqueous humor determines the eye's IOP.

Open angle (also called chronic open angle or primary open angle) is the most common type of glaucoma. With this type, even though the anterior structures of the eye appear normal, aqueous fluid builds within the anterior chamber, causing the IOP to become elevated. Left untreated, this may result in permanent damage of the optic nerve and retina. Eye drops are generally prescribed to lower the eye pressure. In some cases, surgery is performed if the IOP cannot be adequately controlled with medical therapy.

Only about 10% of the population suffers from acute angle closure glaucoma. Acute angle closure occurs because of an abnormality of the structures in the front of the eye. In most of these cases, the space between the iris and cornea is more narrow than normal, leaving a smaller channel for the aqueous to pass through. If the flow of aqueous becomes completely blocked, the IOP rises sharply, causing a sudden angle closure attack.

Secondary glaucoma occurs as a result of another disease or problem within the eye such as: inflammation, trauma, previous surgery, diabetes, tumor, and certain medications. For this type, both the glaucoma and the underlying problem must be treated.

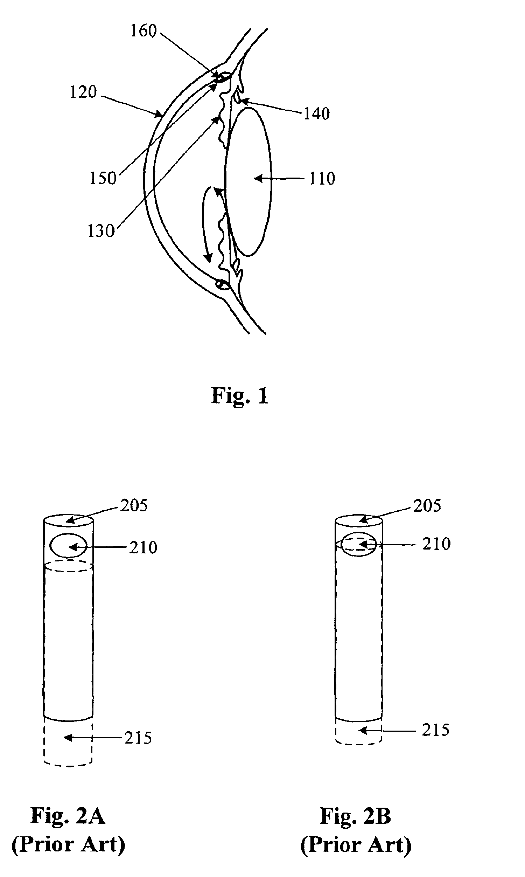

Figure 1 is a diagram of the front portion of an eye that helps to explain the processes of glaucoma. In Figure 1, representations of the lens 110, cornea 120, iris 130, ciliary bodies 140, trabecular meshwork 150, and Schlemm's canal 160 are pictured. Anatomically, the anterior chamber of the eye includes the structures that cause glaucoma. Aqueous fluid is produced by the ciliary bodies 140 that lie beneath the iris 130 and adjacent to the lens 110 in the anterior chamber. This aqueous humor washes over the lens 110 and iris 130 and flows to the drainage system located in the angle of the anterior chamber. The angle of the anterior chamber, which extends circumferentially around the eye, contains structures that allow the aqueous humor to drain. The first structure, and the one most commonly implicated in glaucoma, is the trabecular meshwork 150. The trabecular meshwork 150 extends circumferentially around the anterior chamber in the angle. The trabecular meshwork 150 seems to act as a filter, limiting the outflow of aqueous humor and providing a back pressure producing the IOP. Schlemm's canal 160 is located beyond the trabecular meshwork 150. Schlemm's canal 160 has collector channels that allow aqueous humor to flow out of the anterior chamber. The two arrows in the anterior chamber of Figure 1 show the flow of aqueous humor from the ciliary bodies 140, over the lens 110, over the iris 130, through the trabecular meshwork 150, and into Schlemm's canal 160 and its collector channels.

If the trabecular meshwork becomes malformed or malfunctions, the flow of aqueous humor out of the anterior chamber can be restricted resulting in an increased IOP. The trabecular meshwork may become clogged or inflamed resulting in a restriction on aqueous humor flow. The trabecular meshwork, thus, sometimes blocks the normal flow of aqueous humor into Schlemm's canal and its collector channels.

Surgical intervention is sometimes indicated for such a blockage. Numerous surgical procedures have been developed to either remove or bypass the trabecular meshwork. The trabecular meshwork can be surgically removed by cutting, ablation, or by means of a laser. Several stents or conduits are available that can be implanted through the trabecular meshwork in order to restore a pathway for aqueous humor flow. Each of these surgical procedures, however, has drawbacks.

One approach that does not have the drawbacks of existing procedures involves using a small gauge mechanical tissue cutter/aspirator probe to remove trabecular meshwork tissue. A small gauge cutting device can be guided into Schlemm's canal and moved in a forward motion following the curvature of the trabecular meshwork. The motion causes the trabecular meshwork to be fed into the cutting port of the cutter, cutting and removing the trabecular meshwork blocking the outflow of the aqueous humor.

Document US 4530 359 describes a device acording to the preamble of claim 1.

### SUMMARY OF THE INVENTION

The present invention provides a mechamical tissue cutter/aspirator probe in accordance with claims which follow.

The present invention is a small gauge mechanical tissue cutter/aspirator probe comprising a generally cylindrical first outer cannula, a port located near a distal end of the first outer cannula on a side of the first outer cannula, a second smaller gauge cannula located within first outer cannula connected to a diaphragm that reciprocates the second inner cannula within and along the axis of the first outer cannula, and a retractable pick. A distance between the distal end of the outer cannula and the port is approximately equal to the distance between the back wall of Schlemm's canal and the trabecular meshwork in a human eye.

In another embodiment consistent with the principles of the present invention, the present invention is a small gauge mechanical tissue cutter/aspirator probe comprising a generally cylindrical first outer cannula with a smooth distal end, a port located near a distal end of the first outer cannula on a side of the first outer cannula, a second smaller gauge cannula located within first outer cannula connected to a diaphragm that reciprocates the second inner cannula within and along the axis of the first outer cannula, and a distance between the distal end of the first outer cannula and the port is approximately equal to the distance between the back wall of Schlemm's canal and the trabecular meshwork in a human eye.

It is to be understood that both the foregoing general description and the following detailed description are exemplary and explanatory only and are intended to provide further explanation of the invention as claimed. The following description, as well as the practice of the invention, set forth and suggest additional advantages and purposes of the invention.

### BRIEF DESCRIPTION OF THE DRAWINGS

The accompanying drawings, which are incorporated in and constitute a part of this specification, illustrate several embodiments of the invention and together with the description, serve to explain the principles of the invention.
Figure 1 is a diagram of the front portion of an eye.
Figures 2A and 2B are perspective views of a small gauge mechanical tissue cutter/aspirator probe (traditional vitrectomy probe).
Figure 3 is a perspective view of a small gauge mechanical tissue cutter/aspirator probe according to the principles of the present invention.
Figure 4 is a perspective view of a tapered small mechanical tissue cutter/aspirator probe according to the principles of the present invention.
Figures 5A and 5B are side cross section views of the distal end of an embodiment of a small gauge mechanical tissue cutter/aspirator probe according to the principles of the present invention.
Figures 6A-6C are side cross section views of the distal end of an embodiment of a small gauge mechanical tissue cutter/aspirator probe according to the principles of the present invention.
Figures 7 and 8 are top views of the distal end of various embodiments of a small gauge mechanical tissue cutter/aspirator probe according to the principles of the present invention.
Figures 9 and 10 are views of a small gauge mechanical tissue cutter/aspirator probe as used in glaucoma surgery.

### DETAILED DESCRIPTION OF THE PREFERRED EMBODIMENTS

Reference is now made in detail to the exemplary embodiments of the invention, examples of which are illustrated in the accompanying drawings. Wherever possible, the same reference numbers are used throughout the drawings to refer to the same or like parts.

Figures 2A and 2B are perspective views of a traditional mechanical tissue cutter/aspirator probe (vitrectomy probe). In a typical mechanical tissue cutter/aspirator probe, an outer cannula 205 includes port 210. An inner cannula 21 5 reciprocates in cannula 205. One end of inner cannula 215 is configured so that it can cut tissue when as it enters port 210. As shown in Figures 2A and 2B, inner cannula 215 moves up and down in outer cannula 205 to produce a cutting action. Tissue enters port 210 when the mechanical tissue cutter/aspirator probe is in the position shown in Figure 2A. The tissue is cut as inner cannula 215 moves upward closing off port 210 as shown in Figure 2B. Cut tissue is aspirated through the inner cannula and away from the cutting location. Outer cannula 205 has a generally smooth top surface that can be abutted against eye structures without damaging them. As such, the cutting action, which is located on a side of outer cannula 205, allows the top surface of outer cannula 205 to remain smooth.

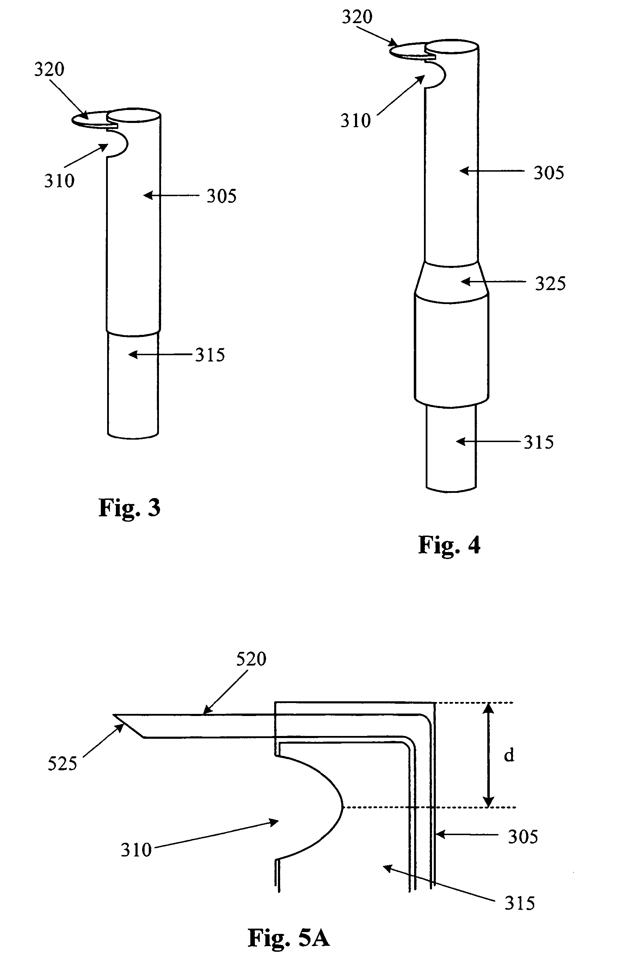

Figure 3 is a perspective view of a small gauge mechanical tissue cutter/aspirator probe according to the principles of the present invention. In the embodiment of Figure 3, an outer cannula 305 includes port 310. An inner cannula 315 reciprocates in outer cannula 305. One end of inner cannula 315 is configured so that it can cut tissue when as it enters port 310. Inner cannula 315 moves up and down in outer cannula 305 to produce a cutting action. Cut tissue can be aspirated through inner cannula 315 and removed from the cutting location. Outer cannula 305 has a generally smooth top surface that can be abutted against eye structures without damaging them. As such, the cutting action, which is located on a side of outer cannula 305, allows the top surface of outer cannula 305 to remain smooth. A retractable pick 320 is located on a distal end of outer cannula 305.

Retractable pick 320 is adapted to fit into Schlemm's canal so that mechanical tissue cutter/aspirator probe cutting action can be used to cut and remove the trabecular meshwork (through aspiration provided through port 310). Retractable pick 320 is a short protrusion that extends outward from the distal tip of outer cannula 305 in the direction of port 310. In one embodiment of the present invention, retractable pick 320 has a sharp end that can be used to pierce the trabecular meshwork so that retractable pick 320 can be placed in Schlemm's canal. While retractable pick 320 facilitates entry into Schlemm's canal, once port 310 is located on the trabecular meshwork, retractable pick 320 is largely unnecessary. As such, retractable pick 320 is retracted into outer cannula 305. Cutting action is provided at port 310 which is located along the trabecular meshwork (as best seen below). The distance between port 310 and the distal end of outer cannula 320 determines the location of port 310 in relation to the back wall of Schlemm's canal. This distance is such that port 310 is located at the trabecular meshwork (preferably the distance from the distal end of outer cannula 305 to the center of port 310 is equal to the distance between the trabecular meshwork and the back wall of Schlemm's canal). Locating port 310 at the trabecular meshwork ensures effective removal of it.

Figure 4 is a perspective view of a tapered small gauge mechanical tissue cutter/aspirator probe according to the principles of the present invention. In this embodiment, the distal end of outer cannula 305 is tapered. While taper 325 is depicted, any type of taper can be employed. Due to the size of Schlemm's canal, it is preferable to have the distal end of outer cannula measure about 0.25 to 0.36 mm diameter (the approximate diameter of Schlemm's canal is about 0.3 mm). In one embodiment, a 27 gauge (0.42mm) cannula is used for outer cannula 305. In other embodiments, a tapered 27 gauge (0.42 mm) or lager cannula is used. Such a cannula is tapered in some fashion so that its distal end measures about 0.25 to 0.36 mm.

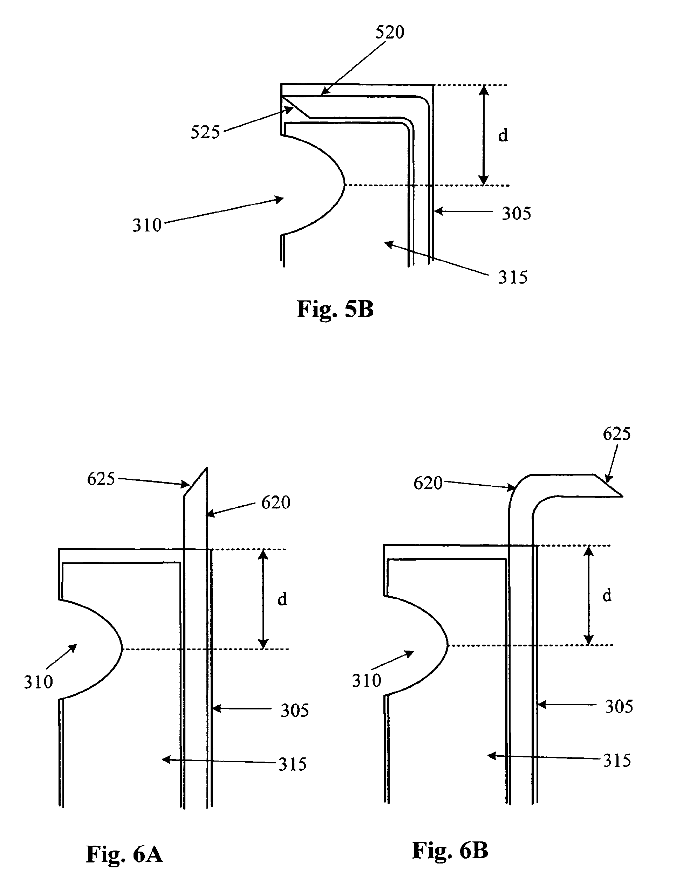

Figures 5A and 5B are side cross section views of the distal end of an embodiment of a small gauge mechanical tissue cutter/aspirator probe according to the principles of the present invention. Figure 5A shows retractable pick 520 in an extended position. Figure 5B shows the retractable pick 520 in a retracted position. In the embodiment of Figure 5A, retractable pick 520 is located at the distal end of cannula 305. Retractable pick 520 may have a sharp tip 525 to pierce the trabecular meshwork so that outer cannula 305 can be properly located for cutting. The distance (d) between the distal end of retractable pick 520 (or the distal end of cannula 305, if retractable pick 520 is not present) is approximately equal to the distance between the back wall of Schlemm's canal and the trabecular meshwork. In this manner, as outer cannula 305 is advanced into Schlemm's canal, the distal end of outer cannula 305 (or retractable pick 520 as the case may be) rests against the back wall of Schlemm's canal so that port 310 is located at the trabecular meshwork.

When retracted, retractable pick 520 is located inside of cannula 305. When extended, retractable pick 520 protrudes through an opening on the outer surface of cannula 305. In one embodiment of the present invention, retractable pick 520 is located between inner cannula 315 and outer cannula 305. Retractable pick 520 travels in a passageway formed between inner cannula 315 and outer cannula 305. In another embodiment of the present invention, a sleeve (not shown) surrounds outer cannula 305. In this case, retractable pick 520 is located between the sleeve (not shown) and the outer cannula 305. Retractable pick 520 travels in a passageway formed between the sleeve (not shown) and outer cannula 305.

Retractable pick 520 may be made of any resilient, durable substance. In one embodiment of the present invention, retractable pick 520 is made of a nitinol wire with a sharpened (or beveled) distal tip. 525. In this case, the sharp tip 525, when extended, can be used to pierce or cut the trabecular meshwork. The sharp tip 525 is then retracted before the outer cannula is placed in Schlemm's canal.

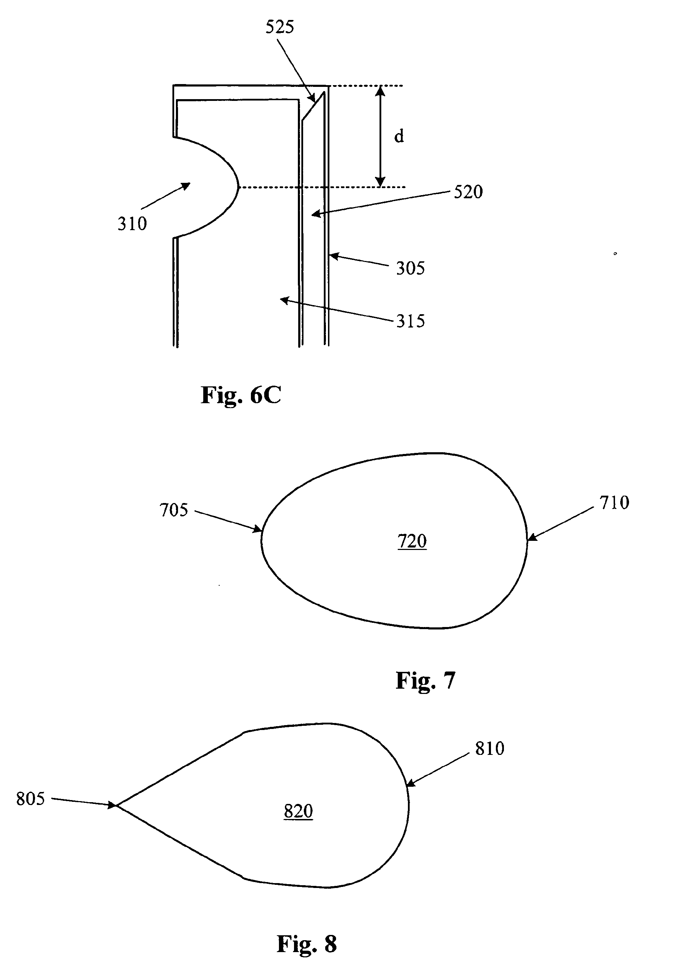

Figures 6A, 6B, and 6C are side cross section views of the distal end of an embodiment of a small gauge mechanical tissue cutter/aspirator probe according to the principles of the present invention. Figures 6A and 6B show retractable pick 620 in an extended position. Figure 6C shows the retractable pick 620 in a retracted position. In the embodiment of Figure 6A, retractable pick 620 is located at the distal end of cannula 305. Retractable pick 620 may have a sharp tip 625 to pierce the trabecular meshwork so that outer cannula 305 can be properly located for cutting. The distance (d) between the distal end of retractable pick 620 (or the distal end of cannula 305, if retractable pick 620 is not present) is approximately equal to the distance between the back wall of Schlemm's canal and the trabecular meshwork. In this manner, as outer cannula 305 is advanced into Schlemm's canal, the distal end of outer cannula 305 (or retractable pick 620 as the case may be) rests against the back wall of Schlemm's canal so that port 310 is located at the trabecular meshwork.

In Figure 6B, retractable pick 620 has a curved profile when in an extended position. In this manner, retractable pick 620 can be oriented with respect to the distal end of cannula 305. In Figure 6A, retractable pick extends outward from the distal end of cannula 305. In Figure 6B, retractable pick extends at an angle from the distal end of cannula 305.

When retracted, retractable pick 620 is located inside of cannula 305. When extended, retractable pick 620 protrudes through an opening on the distal end of cannula 305. In one embodiment of the present invention, retractable pick 620 is located between inner cannula 315 and outer cannula 305. Retractable pick 620 travels in a passageway formed between inner cannula 315 and outer cannula 305. In another embodiment of the present invention, a sleeve (not shown) surrounds outer cannula 305. In this case, retractable pick 620 is located between the sleeve (not shown) and the outer cannula 305. Retractable pick 620 travels in a passageway formed between the sleeve (not shown) and outer cannula 305.

Retractable pick 620 may be made of any resilient, durable substance. In one embodiment of the present invention, retractable pick 620 is made of a nitinol wire with a sharpened (or beveled) distal tip. 625. In this case, the sharp tip 625, when extended, can be used to pierce or cut the trabecular meshwork. The sharp tip 625 is then retracted before the outer cannula is placed in Schlemm's canal. As is commonly known, a nitinol wire retains its shape so as to facilitate the retractable pick arrangement of Figure 6B.

Regardless of what type of pick is used, the distance between the back wall of Schlemm's canal to the trabecular meshwork is about 0.3 mm. The approximate thickness of the trabecular meshwork is 0.1 mm. Accordingly, in the embodiment of the present invention, port 310 has an opening that is greater than 0.1 mm, and the distance from port 310 to the distal tip of cannula 305 is 0.3 mm. In other words, port 310 is located such that it can effectively cut and remove the trabecular meshwork.

Figures 7 and 8 are top views of the distal end of various embodiments of a small gauge mechanical tissue cutter/aspirator probe according to the principles of the present invention. Figures 7 and 8 depict two different embodiments of retractable picks, such as retractable picks 320 or 520. In Figure 7, retractable pick 720 is generally egg shaped with a leading edge 705 and a trailing edge 710. Leading edge 705 extends outward from an outer cannula and is used to pierce the trabecular meshwork. Trailing edge 710 is generally flush with the outer surface of the outer cannula. In the embodiment of Figure 7, leading edge is generally curved and may be sharp or blunt. If leading edge 705 is sharp, it is configured to pierce the trabecular meshwork so that the outer cannula can be advanced into Schlemm's canal and the cutting port can be aligned with the trabecular meshwork. In Figure 8, retractable pick 820 has a point at leading edge 805. Leading edge 805 extends outward from an outer cannula and is used to pierce the trabecular meshwork. Trailing edge 810 is generally flush with the outer surface of the outer cannula. In the embodiment of Figure 8, leading edge is pointed and may be sharp or blunt. If leading edge 805 is sharp, it is configured to pierce the trabecular meshwork so that the outer cannula can be advanced into Schlemm's canal and the cutting port can be aligned with the trabecular meshwork.

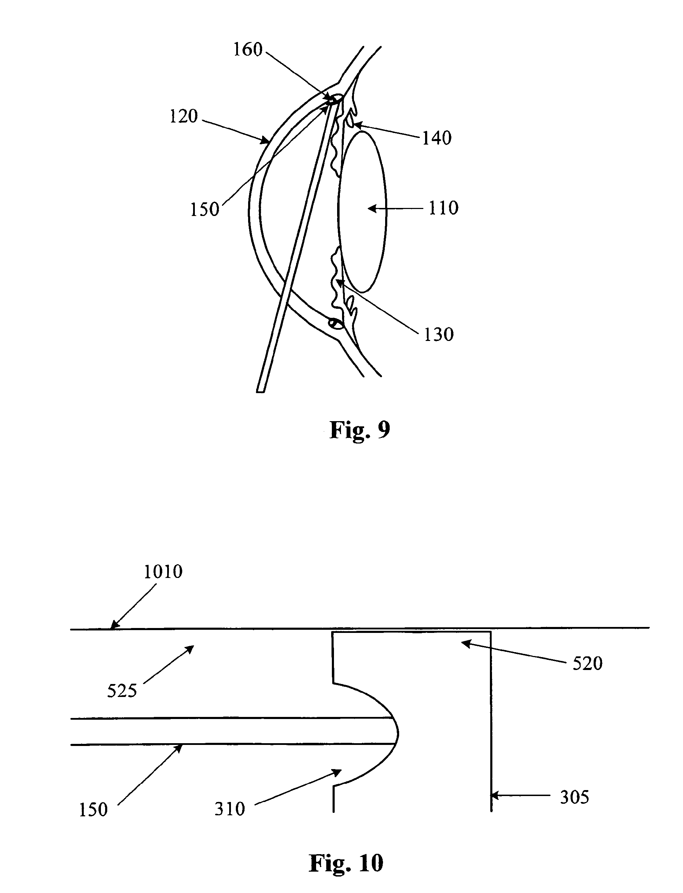

Figures 9 and 10 are views of a small gauge mechanical tissue cutter/aspirator probe as used in glaucoma surgery. In Figure 9, outer cannula 305 is inserted through a small incision in the cornea 120. The distal end of cannula 305 (the end that has port 310) is advanced through the angle to the trabecular meshwork 150. The retractable pick is extended so that an opening can be made in the trabecular meshwork. The retractable pick is then retracted so as to avoid damaging a wall of Schlemm's canal 160. The distal end of cannula 305 is then advanced through the opening in the trabecular meshwork 150 and into Schlemm's canal 160. In this position, port 310 is located at the trabecular meshwork 150 and is ready to be cut and removed from the eye.

Figure 10 is an exploded view of the location of the distal end of outer cannula 305 during the removal of the trabecular meshwork 150 (note that in this position, the retractable pick is in a retracted position). In this position, port 310 is located at the trabecular meshwork 150. Outer cannula 305 is then advanced in the direction of port 310 to cut and remove the trabecular meshwork 150. Outer cannula 305 is advanced through an arc in one direction, port 310 is then rotated 180 degrees, and outer cannula 305 is then advanced in an arc in the other direction. In this manner, the distal end of cannula 305 (and port 310) is moved in an arc around the circumference of the angle to remove a substantial portion of the trabecular meshwork through a single corneal incision. If desired, a second corneal incision opposite the first corneal incision can be made so that the outer cannula 305 can be swept through a second arc of the angle. In this manner, either through one or two corneal incisions, a significant portion of the trabecular meshwork can be cut and removed by the mechanical tissue cutter/aspirator probe.

From the above, it may be appreciated that the present invention provides a system for performing glaucoma surgery with a small gauge mechanical tissue cutter/aspirator probe. The present invention provides a small gauge mechanical tissue cutter/aspirator probe with an optional guide that can be advanced into Schlemm's canal to cut and aspirate the trabecular meshwork. The present invention is illustrated herein by example, and various modifications may be made by a person of ordinary skill in the art.

## Claims

1. A mechanical tissue cutter/aspirator probe comprising:
a generally cylindrical outer cannula (305);
an inner cannula (315) that is slidable in the outer cannula;
a port (310) located near a distal end of the outer cannula; and
a retractable pick (320,520,620,720,820) located on the distal end of the outer cannula so as to extend from the distal end of the outer cannula,
**characterized in that**
the outer cannula has a distal end that defines a generally planar surface, and wherein in use a distance between the generally planar surface of the distal end of the outer cannula and the port is 0.3 millimeters, being equal to the distance between a back wall of Schlemm's canal (160) and a trabecular meshwork (150) in a human eye;
the inner cannula is reciprocable within the outer cannula so as to produce a cutting action, and
wherein the retractable pick is adapted to travel in a passageway formed between the inner cannula (315) and the outer cannula (305), or in a passageway formed between a sleeve surrounding the outer cannula and the outer cannula.

2. The probe of claim 1, wherein the opening of the port (310) is greater than 0.1 millimeters.

3. The probe of claim 2, wherein the retractable pick (320,520,620,720,820) further comprises a sharp edge (525,625,705,805) for piercing the trabecular meshwork.

4. The probe of claim 1, wherein the retractable pick is made of nitinol.

5. The probe of claim 1, wherein, when the retractable pick is adapted to travel in a passageway formed between the inner cannula (315) and the outer cannula (305), the retractable pick (320) is a short protrusion that extends outward from the distal tip of the outer cannula (305) through an opening on the distal end of the outer cannula to one side on which the port (310) is located.

6. The probe of claim 1, wherein the outer cannula (305) is tapered.

7. The probe of claim 1, wherein the outer cannula (305) has a diameter between about 0.25 and 0.36 millimeters.

8. The probe of claim 1, adapted to aspirate the cut tissue through the port (310).

## Patentansprüche

1. Mechanische Gewebeschneide/Aspirationssonde, aufweisend:
eine im Wesentlichen zylindrische Außenkanüle (305);
eine Innenkanüle (315), die in der Außenkanüle gleiten kann;
eine Eingangsmündung (310), die nahe einem distalen Ende der Außenkanüle platziert ist; und
einen rückziehbaren Picker (320, 520, 620, 720, 820), der an dem distalen Ende der Außenkanüle platziert ist, um sich so von dem distalen Ende der Außenkanüle zu erstrecken,
**dadurch gekennzeichnet, dass** die Außenkanüle ein distales Ende hat, das im Wesentlichen eine planare Oberfläche definiert, und wobei bei einer Verwendung ein Abstand zwischen der im Wesentlichen planaren Fläche des distalen Endes der Außenkanüle und der Eingangsmündung von 0,3 mm besteht, der dem Abstand zwischen einer Rückwand des Schlemm's Kanal (160) und einem Trabekelwerk (150) in einem menschlichen Auge entspricht;
dass die Innenkanüle innerhalb der Außenkanüle verschiebbar ist, um so eine Schneidbewegung zu vollziehen, und
wobei der rückziehbare Picker dazu bestimmt ist, in einem Durchgang bewegt zu werden, der zwischen der Innenkanüle (315) und der Außenkanüle (305) gebildet ist, oder in einem Durchgang, der zwischen einer die Außenkanüle umgebenden Buchse und der Außenkanüle gebildet ist.

2. Sonde nach Anspruch 1, wobei die Öffnung der Eingangsmündung (310) größer ist als 0,1 mm.

3. Sonde nach Anspruch 2, wobei der rückziehbare Picker (320, 520, 620, 720, 820) ferner einen scharfen Rand (525, 625, 705, 805) zum Piercen des Trabekelwerkes aufweist.

4. Sonde nach Anspruch 1, wobei der rückziehbare Picker aus Nitinol gefertigt ist.

5. Sonde nach Anspruch 1, wobei der rückziehbare Picker (320) in dem Fall, bei dem der rückziehbare Picker dazu bestimmt ist, in einem zwischen der Innenkanüle (315) und der Außenkanüle (305) gebildeten Durchgang bewegt zu werden, ein kurzer Vorsprung ist, der von dem distalen Ende der Außenkanüle (305) nach außen abragt durch eine Öffnung an dem distalen Ende der Außenkanüle an eine Seite, an der die Eingangsmündung (310) platziert ist.

6. Sonde nach Anspruch 1, wobei die Außenkanüle (305) verjüngt ist.

7. Sonde nach Anspruch 1, wobei die Außenkanüle (305) einen Durchmesser zwischen ca. 0,25 und 0,36 mm hat.

8. Sonde nach Anspruch 1, die dazu bestimmt ist, dass geschnittene Gewebe durch die Eingangsmündung (310) zu aspirieren.

## Revendications

1. Instrument à bistouri mécanique/sonde d'aspiration comprenant :
une canule externe généralement cylindrique (305) ;
une canule interne (315) qui peut coulisser dans la canule externe ;
un orifice (310) positionné à proximité d'une extrémité distale de la canule externe ; et
un pic rétractable (320, 520, 620, 720, 820) situé sur l'extrémité distale de la canule externe de façon à s'étendre à partir de l'extrémité distale de la canule externe,
**caractérisé en ce que** :
la canule externe a une extrémité distale qui définit une surface généralement plane, et dans lequel, à l'usage, une distance entre la surface généralement plane de l'extrémité distale de la canule externe et l'orifice est de 0,3 millimètre, étant égale à la distance entre une paroi arrière d'un canal de Schlemm (160) et un trabéculum cornéo-scléral (150) dans un oeil humain ;
la canule interne peut effectuer un mouvement de va-et-vient à l'intérieur de la canule externe de façon à produire une action coupante, et
dans lequel le pic rétractable est adapté pour se déplacer dans une voie de passage formée entre la canule interne (315) et la canule externe (305), ou dans une voie de passage formée entre un manchon entourant la canule externe et la canule externe.

2. Sonde selon la revendication 1, dans laquelle l'ouverture de l'orifice (310) est supérieure à 0,1 millimètre.

3. Sonde selon la revendication 2, dans laquelle le pic rétractable (320, 520, 620, 720, 820) comprend en outre un bord pointu (525, 625, 705, 805) pour percer le trabéculum coméo-scléral.

4. Sonde selon la revendication 1, dans laquelle le pic rétractable est réalisé avec du nitinol. 1.

5. Sonde selon la revendication 1, dans laquelle, lorsque le pic rétractable est adapté pour se déplacer dans une voie de passage formée entre la canule interne (315) et la canule externe (305), le pic rétractable (320) est une saillie courte qui s'étend vers l'extérieur à partir de la pointe distale de la canule externe (305) par une ouverture sur l'extrémité distale de la canule externe jusqu'à un côté sur lequel l'orifice (310) est situé.

6. Sonde selon la revendication 1, dans laquelle la canule externe (305) est effilée.

7. Sonde selon la revendication 1, dans laquelle la canule externe (305) a un diamètre compris entre environ 0,25 et 0,36 millimètre.

8. Sonde selon la revendication 1, adaptée pour aspirer le tissu coupé par l'orifice (310).
